# EUROPEAN PATENT APPLICATION

(11) **EP 1 958 521 A1**
(43) Date of publication of application: **20.08.2008**
(21) Application number: 06834257.5
(22) Date of filing: 01.12.2006
(51) Int. Cl.: A23L 1/30, A23F 3/00, A23L 2/52, A61K 31/7048, A61K 47/22, A61P 17/18, A61P 39/06

(54) **METHOD OF IMPROVING ABSORBABILITY OF EPIGALLOCATECHIN, FOOD, DRINK AND FOOD/DRINK MATERIAL USING THE SAME AND METHOD OF PRODUCING THE SAME**

(30) Priority: 06.12.2005 JP 2005351770
(71) Applicant: ITO EN, LTD., Tokyo 151-8550 (JP)
(72) Inventor: UNNO, Tomonori, Tokyo 194-0292 (JP); KAKUDA, Takami, Shizuoka 421-0516 (JP); MIYAZAWA, Teruo c/o Division of Bioscience and Biotechnology for Future Bioindustries, Tsutsumidori-amamiyamachi Aoba-ku Sendai, Miyagi 981-8555 (JP); NAKAGAWA, Kiyotaka c/o Division of Bioscience and Biotechnology for Future Bioindustries, sutsumidori-amamiyamachi Aoba-ku Sendai, Miyagi 981-8555 (JP)
(74) Representative: Bauch-Koepe, Katharina Anna
(86) International application number: PCT/JP2006/324502
(87) International publication number: WO 2007/066744

(57) **Abstract**

Provided are a food, a drink and a food/drink material having improved EGCg absorabability, and a method of producing the same, by improving the absorbability of EGCg to be taken in. The contents of caffeine and/or epigallocatechin gallate are regulated so that the ratio by mass of caffeine to epigallocatechin gallate contained in a food, drink, food/drink material or oral ingesta comes close to 0.4.

## Description

### Technical Field

The present invention relates to a method of improving absorbability of epigallocatechin gallate in foods, drinks and food/drink materials which contain catechin compounds, a method of producing a food, a drink or a food/drink material using the same, and further relates to foods, drinks and food/drink materials in which the absorbability of epigallocatechin gallate is improved.

### Background Art

Green tea, which is extracted from green tea leaves using a teapot or the like to be drunk, has recently been spread as a container-packed beverage, and it is therefore easier to drink with such supply than the conventional. Moreover, it is known that green tea contains tea catechin, and it is also reported that tea catechin is effective in preventing heart disease and cancer. As the healthful effect of tea catechin becomes clearer, drinks in which the content of tea catechin is increased have been provided in the marketplace and positive intake of tea catechin is spreading.

Green tea includes 8 types of catechin compounds. Among them, in particular, epigallocatechin gallate (EGCg) having 8 hydroxyl groups per molecule has been studied with expectation for a wide range of bioactivity, based on its antioxidant effect. As a result, it is confirmed that it has a potent activity in fact. In order to obtain the maximum level of effect of EGCg, it is important to take the EGCg continuously. However, the absorbability of EGCg in humans is very low and it is estimated that the rate at which EGCg transfers into the blood is only 1 to 2% of the intake. Therefore, most of the intake is wasted.

As food materials having effects on the efficiency of catechin, the following components have been proposed: 1) components of pepper (which have effects on metabolism of EGCg and increases the amount and time period that EGCg stays in the blood); and 2) certain types of emulsifying agents (which enhance intestinal absorption). On the other hand, as drinks in which the absorbability of catechin to be taken is improved, Japanese patent No. 3403400 discloses a drink obtained by reducing the amount of alcohol precipitable material which is considered as carbohydrates, and Japanese patent Nos. 3638570 and 3638588 below disclose a black tea drink which is blended so that the contents of non-epi type catechins and epi-type catechins satisfy a predetermined relation.

However, the application of the above-described food materials 1) and 2) is limited by their properties such as taste and flavor and function. Accordingly, it is difficult to apply these materials to a wide range of foods and drinks including beverages and other materials.

Moreover, the reduction in the alcohol precipitable material that is described in Japanese patent No. 3403400 is not the matter applicable to a wide range of foods and drinks. In some cases, it is necessary to add a large amount of catechins, and it is thus expected also that the taste and flavor are lost.

Furthermore, the requirement related to the contents of catechins that is described in Japanese patent No. 3638570 or Japanese patent No. 3638588 is the matter that is usually satisfied in green tea drinks that are prepared in ordinary households. Therefore, it seems unlikely that the application of the above requirement to foods and drinks other than container-packed beverages be effective. In addition, these materials relate to black tea drinks. Therefore, it is difficult to widely apply them to other foods and drinks.

### Disclosure of the Invention

An objective of the present invention is to provide a method for improving absorbability of EGCg which can be applied to a wide range of foods and drinks and improve the absorbability of EGCg to be ingested with foods or drinks, as well as foods, drinks and food/drink materials in which the absorbability of EGCg is improved by using the same, and method for producing these matters.

In order to solve the above problems, the present inventors have conducted intensive studies. As a result, they have found that caffeine affects the absorbability of EGCg and has succeeded in completing the present invention.

That is, the present invention reltaes to:
1. a method of improving the absorbability of epigallocatechin gallate, characterized by regulating the contents of caffeine and/or epigallocatechin gallate so that the ratio by mass of caffeine to epigallocatechin gallate contained in an oral ingesta comes close to 0.4;
2. the method of improving the absorbability according to claim 1, wherein the ratio by mass of caffeine to epigallocatechin gallate contained in the oral ingesta is regulated to be in the range of 0.08 to 1.15, and the oral ingesta excludes green tea drinks;
3. the method of improving the absorbability according to claim 1, wherein the ratio by mass of caffeine to epigallocatechin gallate contained in the oral ingesta is regulated to be in the range of 0.12 to 0.85;
4. a food or drink containing caffeine at a ratio by mass of 2.0 or less (except for zero) to epigallocatechin gallate;
5. the food or drink according to claim 4, excluding green tea drinks, wherein the caffeine is contained at a ratio by mass of 0.08 to 1.15 to the epigallocatechin gallate;
6. the food or drink according to claim 4, wherein the ratio by mass of the caffeine to the epigallocatechin gallate is in the range of 0.12 to 0.85;
7. the food or drink according to claim 4, being provided as a green tea drink, wherein the ratio by mass of the caffeine to the epigallocatechin gallate is in the range of 0.18 to 0.70, the content of epigallocatechin gallate is in the range of 0.05 to 0.8 mg/ml;
8. a food/drink material containing caffeine at a ratio by mass of 2.0 or less (except for zero) to epigallocatechin gallate;
9. the food/drink material according to claim 8, in use for a food additive, wherein the ratio by mass of the caffeine to the epigallocatechin gallate is in the range of 0.12 to 0.85;
10. a method of producing a food or drink, characterized by regulating the contents of caffeine and/or epigallocatechin gallate so that the ratio by mass of caffeine to epigallocatechin gallate contained in the food or drink comes close to 0.4;
11. the method of producing a food or drink according to claim 10, wherein the ratio by mass of caffeine to epigallocatechin gallate contained in the food or drink is regulated to be in the range of 0.12 to 0.85, and the food or drink includes green tea;
12. a method of producing a food/drink material, characterized by regulating the contents of caffeine and/or epigallocatechin gallate so that the ratio by mass of caffeine to epigallocatechin gallate contained in the food/drink material comes close to 0.4; and
13. the method of producing a food/drink material according to claim 12, wherein the ratio by mass of caffeine to epigallocatechin gallate contained in the food/drink material is regulated to be in the range of 0.12 to 0.85, and the food/drink material comprises a food additive.

### Brief Description of the Drawings

Fig. 1 is a diagram showing the relationship between the C/E ratio and the amount of EGCg in the blood after oral ingestion in rats.
Fig. 2 is a graph showing the relationship between the C/E ratio and the amount of EGCg in the blood after oral ingestion in humans.

### Best Mode for Carrying out the Invention

According to one aspect of the present invention, a summary of a method of improving the absorbability of epigallocatechin gallate resides in regulating the contents of caffeine and/or epigallocatechin gallate so that the ratio by mass of caffeine to epigallocatechin gallate contained in oral ingesta comes close to 0.4.

Moreover, according to one aspect of the present invention, a summary of a food or drink resides in containing caffeine at a ratio by mass of 2.0 or less (except for zero) to epigallocatechin gallate.

Furthermore, according to one aspect of the present invention, a summary of a food/drink material resides in containing caffeine at a ratio by mass of 2.0 or less (except for zero) to epigallocatechin gallate.

According to one aspect of the present invention, a summary of a method of producing a food or drink resides in regulating the contents of caffeine and/or epigallocatechin gallate so that the ratio by mass of caffeine to epigallocatechin gallate contained in the food or drink comes close to 0.4.

According to one aspect of the present invention, a summary of a method of producing a food/drink material resides in regulating the contents of caffeine and/or epigallocatechin gallate so that the ratio by mass of caffeine to epigallocatechin gallate contained in the food/drink material comes close to 0.4.

Examples of catechin compounds (total catechin) include four kinds of epi-type catechins (epicatechin (EC), epigallocatechin gallate (ECg), epigallocatechin (EGO) and epigallocatechin gallate (EGCg)) and four kinds of non-epi type catechins (catechin (C), gallocatechin (GC), catechin gallate (Cg) and gallocatechin gallate (GCg)). The catechin compounds contained in tea leaves are four kinds of epi-type catechins and slight amounts of two non-epi type catechins (catechin and gallocatechin). The catechin compound in the tea infusion which is extracted from tea leaves by general preparation methods is mainly epi-type catechin. In this regard, when the epi-type catechin is exposed to high temperatures such as heat sterilization, it is isomerized to the non-epi type catechin. Thus, the content of the non-epi-type catechin is increased in the tea drinks processed into container-packed beverage products.

It is said that EGCg is the highest functional component of catechin compounds, absorption of EGCg into the living body is very low, and the rate at which the ingested EGCg transfers into the blood is 1 to 2% of the amount ingested. However, this is a result of the case of drinking the tea infusion containing various compounds extracted from tea leaves. The present inventors have examined effects of other compounds than catechins on absorption of EGCg, and they have then found that the absorbability of EGCg is changed by the presence of methyl xanthine compound. Examples of the methyl xanthine compound include caffeine, theophylline and theobromine, and the methyl xanthine contained in teas is mainly caffeine. In other words, the absorbability of ECCg varies depending on the amount of caffeine contained in the tea infusion. More particularly, if the ratio of the caffeine content to the ECCg content (hereinafter referred to as C/E ratio) is lower than that ratio in normal tea infusion, the absorbability of EGCg becomes higher. An optimal C/E ratio that maximizes the absorbability of EGCg exists in the range of C/E ratio between zero and the values of normal tea liquid. Therefore, the absorbability of EGCg to be ingested, namely, the amount to be transferred into the blood is increased by adjusting the composition so that the C/E ratio comes close to the optimal value. It is possible to optimize the C/E ratio by regulating the caffeine content and/or the EGCg content. In view of the fact that excessive amounts of catechin compounds impair the taste and flavor of food and drink, suiting to an appropriate C/E ratio where the absorbability may be improved by decreasing the amount of caffeine is very advantageous from the viewpoint that the effect on the taste and flavor of food and drink can be reduced, and it can be applied to a wide range of food and drink.

When changes in the blood concentration (the amount to be transferred into the blood) over time are examined for EGCg and caffeine orally ingested by humans, both concentrations of EGCg and caffeine increase rapidly after the ingestion and they reach a maximum value at about 1.5 hours. Thereafter, they are decreased. Therefore, the absorbability of EGCg can be evaluated by the blood concentration of EGCg after a certain period of time from when it is orally ingested. In examination of the absorbability of EGCg by changing the ingestion amounts of EGCg and caffeine, the absorbability of EGCg in rats increases as the C/E ratio increases from zero, and it decreases at the C/E ratio exceeding 0.2. Then the absorbability of EGCg becomes lower than that of EGCg alone (the C/E ratio = 0) at the C/E ratio being around 0.5. In other words, the absorbability of EGCg reaches the maximum level at the C/E ratio being around 0.2, and it is considered that the absorption of EGCg is inhibited by caffeine when the C/E ratio is about 0.5 or more. Also in the case of humans, the tendency similar to the case of rats is observed in the relationship between the C/E ratio and the absorbability of EGCg. That is, the absorbability of EGCg increases as the C/E ratio of drinks increases from zero, and it turns to decrease when the C/E ratio exceeds 0.4, coming to approximately equal to the absorbability of EGCg alone at the C/E ratio of around 2.0. In this regard, the absorbability of EGCg reaches the maximum level at the C/E ratio of around 0.4 (about 0.3 to 0.5). When the C/E ratio exceeds 1, the difference from the case of EGCg alone is small, however, the absorption inhibition is not observed. That is, the absorbability of EGCg in humans clearly increases when the C/E ratio is about 2.0 or less, and the absorbability of EGCg significantly increases when the C/E ratio is in the range of 0.08 to 1.15.

Consequently, in the case where caffeine and EGCg are contained in the food or drink so that the C/E ratio is about 2.0 or less (except for zero), preferably 0.08 to 1.15, the absorbability of EGCg by ingestion of food or drink increases, which is more significant than that of EGCg alone. Therefore, in production process of usual foods and drinks, the caffeine content and/or the EGCg content is adjusted so that the C/E ratio in food or drink comes close to 0.4, thereby obtaining a food or drink containing EGCg in which the absorbability is improved. Specifically, caffeine and EGCg may be added taking into consideration the amounts of caffeine and EGCg that are originally contained in the food or drink.

With reference to green tea which has the highest catechin content of teas, the contents of caffeine and EGCg that are contained in the infusion extracted by immersing the tea leaves in boiling water vary depending on the temperature of the boiling water. However, it is reported that these values are about 0.21 mg/ml and about 0.24 mg/ml, respectively, when the tea is prepared by a general method (see reference, Takahashi et al., "Investigation of Daily Conditions of Green Tea Drinking and Catechins Intake in Saitama Prefecture", Chagyou Kenkyu Houkoku, 97(2004), 49-58). Based on this reference, the C/E ratio of normal green tea infusion is about 0.9. When the temperature of the hot water is low, the caffeine infusion is reduced and the amount of EGCg is also decreased at the same time. Thus, it is considered that the C/E ratio of general green tea infusion is approximately 0.9. In the case of a product processed into a container-packed beverage, a reduction in the amount of EGCg is caused by thermal sterilization, resulting in increase of the C/E ratio more. Also in the case of fermented tea and semi-fermented tea, the C/E ratio is increased more. Therefore, the C/E ratio of general tea infusion is higher than the optimal C/E ratio where the absorbability of EGCg reaches the maximum level. In order to obtain a tea drink with the optimal C/E ratio from general green tea infusion, the C/E ratio is decreased to be close to 0.4. Examples of the method to achieve this include a method of decreasing the amount of caffeine (decaffeination) and a method of increasing (adding) the amount of EGCg.

In the preparation of a container-packed tea beverage, the infusion extracted from tea leaves is possibly subjected to either of decaffeination or addition of EGCg. In contrast, in the case of beverages other than tea such as health drink and beverage for specified health use, the beverages is possibly prepared by appropriately blending caffeine and EGCg. Taking astringency of EGCg into consideration, a suitable content of EGCg in a drink is 1.0 mg/ml or less in order to give the drink a good taste and flavor, and, it is preferred that the content may be adjusted to about 0.025 to 0.80 mg/ml, more preferably about 0.05 to 0.75 mg/ml. In accordance with the above ranges, the caffeine content corresponding to the appropriate C/E ratio may be calculated, and the caffeine content determined is about 1.15 mg/ml or less, preferably about 0.002 to 0.92 mg/ml, more preferably about 0.004 to 0.86 mg/ml. If necessary, various additives to be used in preparation of common drink such as antioxidants and stabilizing agents, flavoring agents, spices and the like may be appropriately blended to the drinks according to the present invention. Acidic substances such as ascorbic acid, citric acid, etc. have effect to prevent EGCg from decreasing due to non-epimerization at high temperatures and effectively stabilize the beverages at the time of heat sterilization.

The decaffeination of tea infusion can be carried out, for example, by chromatography using a column filled with a synthetic adsorbent (Japanese Patent No. 3403400, paragraph [0027], etc.). In the present invention, it is not necessary to completely remove caffeine, and the removal rate in the decaffeination can be adjusted properly depending on the caffeine content that is required for the final products. Alternatively, the ratio of EGCg to be added may be adjusted in accordance with the caffeine removal rate. In addition of EGCg, the EGCg to be used may be a purified one or a composition containing other catechin compounds and the like obtained from tea extracts. According to the reference, the presence of catechins other than EGCg does not substantially affect the absorbability of EGCg (See: Henning et al., "Bioavailability and antioxidant effect of epigallocatechin gallate administered in purified form versus as green tea extract in healthy individuals", Journal of Nutritional Biochemistry, 16 (2005), p610-616). For example, commercially available catechin formulations ("Thea-Flan", manufactured by Ito En, Ltd.; "Teavigo", manufactured by DSM Nutritional Products, etc.), concentrates obtained after decaffeinating the tea infusion and the like are actually usable.

If the drink (or a raw material liquid of the drink) optimized so that the C/E ratio comes close to 0.4 as described above is concentrated or dried to prepare concentrate or dry substance, it is possibly used as a powdered drink or instant tea. Alternatively, it may also be used as a material for food and drink, and it is available as a constituent of, for example, supplement and complementary food. Moreover, the above food/drink material may be used as a food additive that is added to various foods and drinks, and it makes possible to add EGCg to foods and drinks other than green tea drink under conditions where the absorbability is improved. Accordingly, it is made possible to enjoy the efficacy of improvement in the absorbability of EGCg in various foods and drinks. The food additive may also be prepared by mixing purified caffeine and purified EGCg at the preferable C/E ratio, and flavoring agents, spices, pH adjustors, thickeners and the like which are available for general use, may be added thereto. The food additive can be prepared in various forms such as powder, solid, liquid, paste and the like. The addition of acidulants is effective in reducing the pH so as to prevent the isomerization of the epi-type catechin. The food additive as described above can be applied to various foods and drinks such as beverages, confectioneries, daily dishes, instant foods, processed foods and the like. Food and drink containing EGCg in which the absorbability is improved are provided with use of the food additive during or after the production process of food and drink. Moreover, a proper tea flavor can be added according to the addition amount thereof. It may also be added to the raw material for processing food and drink to improve the absorbability of EGCg, and it can be give for example to add to the raw material such as grain flours that include wheat flour and buckwheat flour; flavoring agents that include sugar, salt, soy sauce, bean paste (Miso) and vinegar, and the like.

If the above-described optimization of the C/E ratio is applied to tea leaf products, the absorbability of EGCg in tea drinks that are prepared in ordinary households can be improved. Specifically, a decaffeinated green tea extract or a catechin formulation is attached to tea leaves to adjust the C/E ratio of the whole tea leaf product so that the C/E ratio of tea drinks obtained from the tea leaves is the suitable value as described above. In this connection, if low-caffeine tea leaves (tea leaves in which caffeine is reduced) which are produced by soaking steamed leaves in boiling water are used as the tea leaves for the main material, it is easy to adjust the C/E ratio and taste and flavor. Since the catechin content of fermented tea or semi-fermented tea such as black tea and oolong tea is lower than that of green tea, application of the present invention possibly contributes to the effective intake of EGCg.

Thus the present invention makes possible to improve the absorption of EGCg in a wide range of foods and drinks and allow a health-effective amount of EGCg to be easily taken in even from food and drink with a low content of EGCg. It is therefore useful for the improvement of health.

In the case of using the above-described food additive with the suitable C/E ratio, if the food or drink to be applied contains caffeine, the C/E ratio may depart from a suitable scope. Therefore, it is desirable that, when the food or drink (applications) to be applied is selected in advance, the C/E ratio of food additive is determined in view of the caffeine content of that food or drink and the ratio of the standard addition. Examples of such food and drink include caffeine-containing beverages such as coffee, cocoa (chocolate drink) and the like, and foods and drinks using the same. In the present invention, a low-caffeine coffee, cocoa, chocolates, and foods and drinks using the same are advantageously used to provide food and drink of a high level both in the EGCg content and absorbability.

Moreover, the food additive with a suitable C/E ratio according to the present invention may be used for foods and drinks that are made from a raw material without caffeine, such as grains, vegetables, fruits and the like. In that case, the amount of food additive can be appropriately changed within the range that keeps a good taste and flavor.

The C/E ratio of normal green tea drink is about 0.9. Thus, if the C/E ratio is adjusted to less than 0.9, specifically 0.12 to 0.85, the absorbability of EGCg is clearly improved in comparison with the normal green tea infusion. When the C/E ratio is 0.18 to 0.70, the absorbability of EGCg is equal to or more than about 1.1 times the absorbability of normal green tea infusion (that is, equal to or more than about 1.7 times the absorbability of EGCg alone). When the C/E ratio is 0.20 to 0.60, the absorbability of EGCg is equal to or more than about 1.15 times the absorbability of normal green tea infusion (that is, equal to or more than about 1.75 times the absorbability of EGCg alone). Therefore, a practical effect of the increase of the absorbability can be obtained well.

According to the reference, it is reported that, in the case where green tea intake per day which is effective in preventing cancer is determined based on the conventional green tea drinks, it is about 10 cups of green tea (≈1.5 L, the amount of EGCg to be contained: 360 to 540 mg) (Nakachi, et al., "Preventive effects of drinking green tea on cancer and cardiovascular disease: Epidemiological evidence for multiple targeting prevention", BioFactors, 13 (2000), 49-54). On the basis of this fact, in the present invention which can increase the absorbability of EGCg up to 1.2 times that of normal tea, the effective intake of green tea per day is 8.3 cups (1.25 L) in the case of green tea with the EGCg content at the same level as that of general tea infusion (about 0.24 mg/ml). If the amount of EGCg is increased within the range that can minimize the effect on the taste and flavor and if the C/E ratio is adjusted in the range of the EGCg content higher than 0.24 mg/ml, the effective intake per day can be decreased to 5 cups or less.

### EXAMPLES

Herein below, the improvement in the absorbability of EGCg according to the present invention will be specifically described with reference to Examples.

### Example 1

### (Preparation of samples)

Solutions obtained respectively by dissolving 0 to 65 mg of a caffeine reagent (manufactured by Wako Pure Chemical Industries, Ltd.) in 3 ml of water and a solution obtained by dissolving 30 mg of EGCg formulation (content of EGCg: 90% or more, trade name: Teavigo, manufactured by DSM Nutritional Products) in 1 ml of water were prepared.

### (Absorbability of EGCg in rats)

In accordance with Experimental plots 1 to 7 shown in Table 1, the solution of caffeine and the solution of EGCg were orally administered to rats. After 60 minutes from the administration, the blood EGCG level in rats was measured by chemiluminescence-HPLC. The measurement was subjected by using six rats for each Experimental plot. The average of the values measured for six rats in each plot is shown in Table 1.

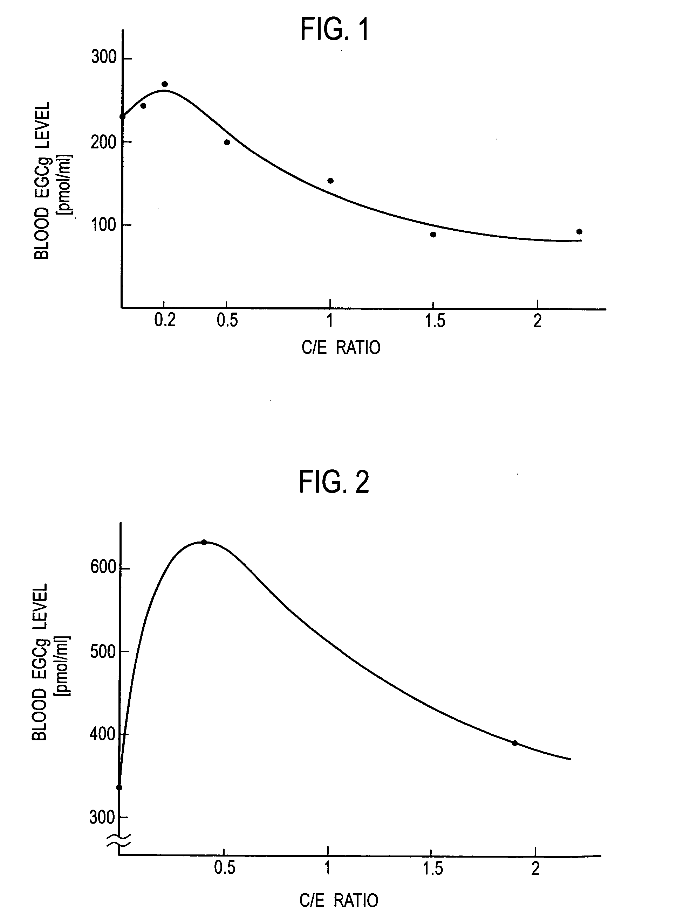

The relationship between the C/E ratio and the blood EGCg level is graphically represented in accordance with Table 1, which is shown as FIG. 1. According to Table 1, the blood EGCg level in rats is the highest when the C/E ratio is 0.2 in Experimental plot 3. When the C/E ratio is lower or higher than that, the blood EGCg level is reduced. Moreover, in Experimental plots 4 to 7, the blood EGCg level is lower than that of Experimental plot 1 where caffeine is not administered. These results show that the absorption of EGCg is inhibited by caffeine when the C/E ratio is about 0.5 or more. It is demonstrative from this point that the C/E ratio being less than 0.5 is important for improving the absorbability of EGCg.

**(Table 1)**

| | Caffeine | EGCg | C/E ratio | Blood EGCg level |
|---|---|---|---|---|
| | (mg) | (mg) | | (pmol/ml) |
| Exp. plot 1 | 0 | 30 | 0 | 229 |
| Exp. plot 2 | 3 | 30 | 0.1 | 243 |
| Exp. plot 3 | 6 | 30 | 0.2 | 270 |
| Exp. plot 4 | 15 | 30 | 0.5 | 221 |
| Exp. plot 5 | 30 | 30 | 1.0 | 153 |
| Exp. plot 6 | 45 | 30 | 1.5 | 90 |
| Exp. plot 7 | 65 | 30 | 2.2 | 94 |

### Example 2

### (Preparation of drinks)

Caffeine formulation (manufactured by SHIRATORI Pharmaceutical Co., Ltd.), EGCg formulation (content of EGCg: 90% or more, trade name: Teavigo, manufactured by DSM Nutritional Products), ascorbic acid and citric acid were added in the amounts shown in Table 2, respectively, to 190 ml of water, which were dissolved. Thereafter, each of the resulting mixtures was poured into a can, respectively, which was sealed and sterilized by heat and then drinks A to C were prepared. The actual values of the contents of caffeine and catechin in the prepared drinks are shown in the table.

### (Absorbability of EGCg in humans)

Each of subjects drank one of the drinks A to C, and the blood EGCG level at 90 minutes after the drinking was measured by chemiluminescence-HPLC. The measurement was subjected in three subjects for each drink. The average of the values measured for three subjects are shown in Table 2.

The relationship between the C/E ratio and the blood EGCg level is graphically represented in accordance with Table 2, which is shown as FIG. 2. According to Table 2, the blood EGCg level of subjects is the highest in the case that the drink B having the C/E ratio of 0.4 has been taken by the subjects, and the blood EGCg level falls in either case where the C/E ratio is lower or higher than that. In the case of drink C, the blood EGCg level is higher than that of drink A containing no caffeine, and, according to this result, inhibition of EGCg absorption by caffeine is not observed in the range where the C/E ratio exceeds about 0.4. It is clear that an optimal C/E ratio in which the absorbability of EGCg increases most significantly is in the range of 0.6 or less.

**(Table 2)**

| | Caffeine | EGCg | Ascorbic acid | Citric acid | C/E ratio | Blood EGCg level |
|---|---|---|---|---|---|---|
| | (mg) | (mg) | (mg) | (mg) | | (pmol/ml) |
| Drink A | 0 | 94 | 24 | 4 | 0 | 336 |
| Drink B | 41 | 95 | 23 | 4 | 0.4 | 631 |
| Drink C | 180 | 94 | 22 | 4 | 1.9 | 390 |

### Industrial Applicability

According to the present invention, the ratio of caffeine to EGCg is optimized to improve the absorption of EGCg so that the EGCg to be orally ingested is absorbed into the body at a higher level than that of usual foods or drinks. This optimization can be applied to production and processing of a wide range of foods, drinks and food/drink materials. Accordingly, foods, drinks and food/drink materials in which the absorbability of EGCg is improved are provided. The food/drink material in which the absorbability of EGCg is improved is used as a food additive, to make easy to produce and provide foods and drinks in which the absorbability of EGCg is improved.

## Claims

1. A method of improving the absorbability of epigallocatechin gallate, **characterized by** regulating the contents of caffeine and/or epigallocatechin gallate so that the ratio by mass of caffeine to epigallocatechin gallate contained in an oral ingesta comes close to 0.4.

2. The method of improving the absorbability according to claim 1, wherein the ratio by mass of caffeine to epigallocatechin gallate contained in the oral ingesta is regulated to be in the range of 0.08 to 1.15, and the oral ingesta excludes green tea drinks.

3. The method of improving the absorbability according to claim 1, wherein the ratio by mass of caffeine to epigallocatechin gallate contained in the oral ingesta is regulated to be in the range of 0.12 to 0.85.

4. A food or drink containing caffeine at a ratio by mass of 2.0 or less (except for zero) to epigallocatechin gallate.

5. The food or drink according to claim 4, excluding green tea drinks, wherein the caffeine is contained at a ratio by mass of 0.08 to 1.15 to the epigallocatechin gallate.

6. The food or drink according to claim 4, wherein the ratio by mass of the caffeine to the epigallocatechin gallate is in the range of 0.12 to 0.85.

7. The food or drink according to claim 4, being provided as a green tea drink, wherein the ratio by mass of the caffeine to the epigallocatechin gallate is in the range of 0.18 to 0.70, and the content of epigallocatechin gallate is in the range of 0.05 to 0.8 mg/ml.

8. A food/drink material containing caffeine at a ratio by mass of 2.0 or less (except for zero) to epigallocatechin gallate.

9. The food/drink material according to claim 8, in use for a food additive, wherein the ratio by mass of the caffeine to the epigallocatechin gallate is in the range of 0.12 to 0.85.

10. A method of producing a food or drink, **characterized by** regulating the contents of caffeine and/or epigallocatechin gallate so that the ratio by mass of caffeine to epigallocatechin gallate contained in the food or drink comes close to 0.4.

11. The method of producing a food or drink according to claim 10, wherein the ratio by mass of caffeine to epigallocatechin gallate contained in the food or drink is regulated to be in the range of 0.12 to 0.85, and the food or drink includes green tea.

12. A method of producing a food/drink material, **characterized by** regulating the contents of caffeine and/or epigallocatechin gallate so that the ratio by mass of caffeine to epigallocatechin gallate contained in the food/drink material comes close to 0.4.

13. The method of producing a food/drink material according to claim 12, wherein the ratio by mass of caffeine to epigallocatechin gallate contained in the food/drink material is regulated to be in the range of 0.12 to 0.85, and the food/drink material comprises a food additive.
